# EUROPEAN PATENT APPLICATION

(11) **EP 4 682 534 A1**
(43) Date of publication of application: **21.01.2026**
(21) Application number: 23928506.7
(22) Date of filing: 17.03.2023
(51) Int. Cl.: G01N 33/48, G01N 33/53

(54) **PATHOLOGICAL DIAGNOSIS ASSISTANCE DEVICE, PATHOLOGICAL DIAGNOSIS ASSISTANCE METHOD, AND RECORDING MEDIUM**

(71) Applicant: NEC Corporation, 108-8001 Tokyo (JP)
(72) Inventor: SANO, Maki, Tokyo 108-8001 (JP); AMAKAWA, Ayaka, Tokyo 108-8001 (JP)
(74) Representative: Betten & Resch
(86) International application number: PCT/JP2023/010570
(87) International publication number: WO 2024/194921

(57) **Abstract**

In a pathology diagnosis support device, an image acquisition means acquires a pathological image of a patient. A ROI acquisition means acquires a region of interest which is selected by a user from the pathological image. A search means searches, from the pathological image, for other regions having features similar to features included in the region of interest. An output means outputs results of the search.

## Description

### TECHNICAL FIELD

The present disclosure relates to pathology diagnosis support using pathological image analysis.

### BACKGROUND ART

In a case of performing a pathology diagnosis, an expert such as a pathologist may find an important region from a pathology specimen or a WSI (Whole Slide Images). In such case, the expert may desire to find a region similar to the important region from the entire region of the pathology specimen. For instance, a spatial gene expression analysis is known, which allows gene expression analysis while maintaining positional information in tissue sections, and the expert may desire to efficiently perform a spatial genetic information analysis using only the important region and the region similar to the important region in the pathology specimen. In addition, the expert may desire to overlap spatial genetic information with the pathology specimen, discover genomic mutations from the spatial genetic information, and confirm the region similar to a cell image or a tissue image where genomic mutations were discovered from the pathology specimens. However, it is difficult to find the region similar to the important region from the entire region of the pathology specimen, and an original important region may be lost while searching. Patent Document 1 describes a pathology diagnosis support device that, in a case of observing a pathological image, searches for and displays case images including a portion similar to a predetermined region of the pathological image from accumulated diagnosed case images.

### PRECEDING TECHNICAL REFERENCES

### PATENT DOCUMENT

Patent Document 1: Japanese Laid-open Patent Publication No. WO2014-103664

### SUMMARY

### PROBLEM TO BE SOLVED BY THE INVENTION

However, even with a method of Patent Literature 1, it is not always possible to search for regions similar to an important region from the entire region of a pathology specimen.

It is one object of the present disclosure to provide a pathology diagnosis support device, a pathology diagnosis support method, and a recording medium capable of searching for regions in a pathological image that have similar features to a predetermined region.

### MEANS FOR SOLVING THE PROBLEM

According to an example aspect of the present disclosure, there is provided a pathology diagnosis support device including:
an image acquisition means configured to acquire a pathological image of a patient;
an ROI acquisition means configured to acquire a region of interest which is selected by a user from the pathological image;
a search means configured to search, from the pathological image, for other regions having features similar to features included in the region of interest; and
an output means configured to output results of the search,
wherein the search means performs a search for one or more regions of a similar size to the region of interest as search targets, for each type of feature included in the pathological image,
the output means outputs one or more results of the search for each type of feature, and
the results of the search include a reason for the search and information of a search source.

According to another example aspect of the present disclosure, there is provided a pathology diagnosis support method including:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.

According to a further example aspect of the present disclosure, there is provided a recording medium storing a program, the program causing a computer to perform a process including:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.

### EFFECT OF THE INVENTION

According to the present disclosure, it is possible to search for regions in a pathological image that have similar features to a predetermined area.

### BRIEF DESCRIPTION OF THE DRAWINGS

[FIG. 1] FIG. 1 illustrates a pathology diagnosis support device according to a first example embodiment.
[FIG. 2] FIG. 2 is a block diagram illustrating a hardware configuration of the pathology diagnosis support device according to the first example embodiment.
[FIG. 3] FIG. 3 is a block diagram illustrating a functional configuration of the pathology diagnosis support device according to the first example embodiment.
[FIG. 4] FIG. 4 illustrates an example of a display of search results by the pathology diagnosis support device according to the first example embodiment.
[FIG. 5] FIG. 5 illustrates another example of a display of search results by the pathology diagnosis support device according to the first example embodiment.
[FIG. 6] FIG. 6 illustrates an example of a display of a search screen by the pathology diagnosis support device according to the first example embodiment.
[FIG. 7] FIG. 7 illustrates another example of a display of search results by the pathology diagnosis support device according to the first example embodiment.
[FIG. 8] FIG. 8 is a flowchart of data output processing by the pathology diagnosis support device according to the first example embodiment.
[FIG. 9] FIG. 9 illustrates a display example of Modification 1 according to the first example embodiment.
[FIG. 10] FIG. 10 illustrates an example of an input screen for a search target image according to a second example embodiment.
[FIG. 11] FIG. 11 illustrates an example of a display of search results by a pathology diagnosis support device according to the second example embodiment.
[FIG. 12] FIG. 12 illustrates an example of a display of a search screen by the pathology diagnosis support device according to the second example embodiment.
[FIG. 13] FIG. 13 illustrates another example of a display of search results by the pathology diagnosis support device according to the second example embodiment.
[FIG. 14] FIG. 14 is a block diagram illustrating a functional configuration of the pathology diagnosis support device according to a third example embodiment.
[FIG. 15] FIG. 15 is a flowchart of processing by the pathology diagnosis support device according to the third example embodiment.

### EXAMPLE EMBODIMENTS

In the following, example embodiments will be described with reference to the accompanying drawings.

### <First Example Embodiment >

### [System Configuration]

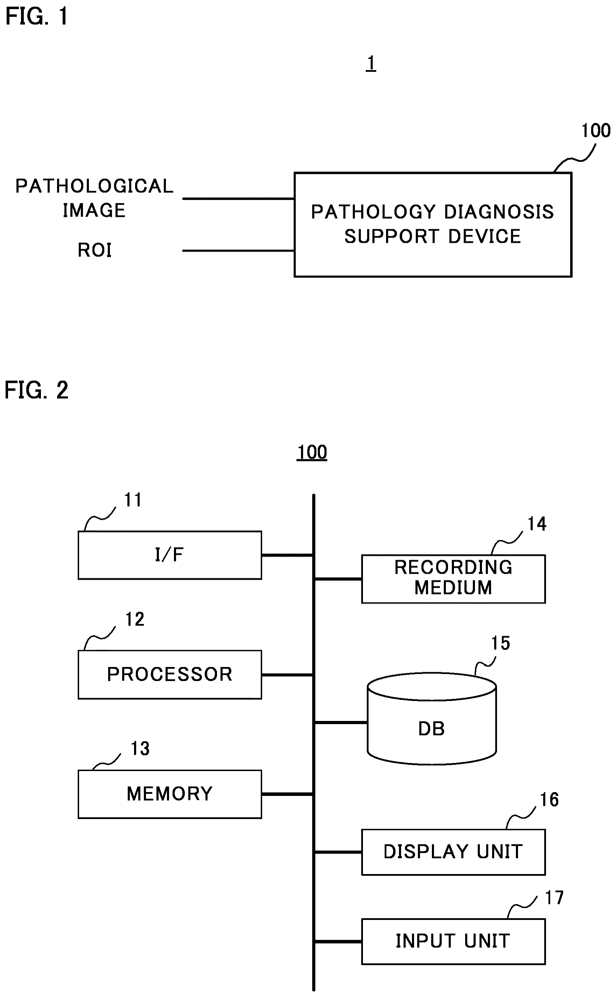

FIG. 1 illustrates a pathology diagnosis support device according to a first example embodiment. A pathology diagnosis support device 100 in a system 1 searches for and displays other regions similar to an ROI (Region of Interest) from a pathological image based on input data of the pathological image and the ROI for the pathological image. Note that the ROI in this example embodiment refers to a region that a user desires to search. For instance, in a case where the user finds an important region during an observation of the pathological image, the user designates the important region as the ROI. This allows the user to easily find other regions similar to the important region from the entire region of the pathology specimen.

### [Hardware Configuration]

FIG. 2 illustrates a hardware configuration of the pathology diagnosis support device 100. The pathology diagnosis support device 100 mainly includes an interface (I/F) 11, a processor 12, a memory 13, a recording medium 14, a database (hereinafter referred to as "DB") 15, a display unit 16, and an input unit 17.

The I/F 11 performs input/output of data with an external device. The pathological images and the ROI are input through the I/F 11.

The processor 12 is a computer such as a CPU (Central Processing Unit) and controls the entire pathology diagnosis support device 100 by executing a program prepared in advance. Note that the processor 12 may be a GPU (Graphics Processing Unit) or an FPGA (Field-Programmable Gate Array).

The memory 13 is composed of a ROM (Read Only Memory), a RAM (Random Access Memory), and the like. The memory 13 is also used as a working memory during executions of various processes by the processor 12.

The recording medium 14 is a non-volatile and non-transitory recording medium such as a disk-shaped recording medium or a semiconductor memory, and is configured to be removably attached to the pathology diagnosis support device 100. The recording medium 14 records various programs executed by the processor 12. In a case where the pathology diagnosis support device 100 executes the various processes, corresponding programs recorded in the recording medium 14 are loaded into the memory 13 and executed by the processor 12.

The DB 15 stores each search result of the pathology diagnosis support device 100 as needed. The DB 15 may include an external storage device such as a hard disk connected to or built into the pathology diagnosis support device 100, and may include a removable storage medium such as a flash memory. Note that instead of providing the DB 15 within the pathology diagnosis support device 100, the DB 15 may be provided on an external server or the like, and each search result may be stored on the server via communication.

The display unit 16 is, for instance, a liquid crystal display device, and displays a search result by the pathology diagnosis support device 100. The input unit 17 is, for instance, a mouse, a keyboard, or the like, and is used by the user to give instructions or input.

### [Functional Configuration]

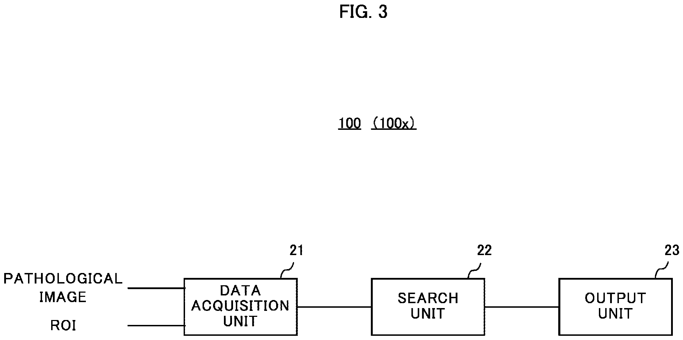

FIG. 3 is a block diagram illustrating a functional configuration of the pathology diagnosis support device 100. The pathology diagnosis support device 100 functionally includes a data acquisition unit 21, a search unit 22, and an output unit 23.

The pathological image and the ROI are input to the pathology diagnosis support device 100 via the I/F 11. The data acquisition unit 21 acquires each pathological image and the ROI. In this example embodiment, as the pathological image, a pathological image obtained from serial sections of a tissue specimen (hereinafter also referred to as a "serial section pathological image specimen") is used. The ROI is designated by the user from the pathological image. The user can freely designate the size and shape of the ROI. For instance, the user can designate an ROI of a single cell nucleus size in a free shape such as a rectangle or a circle. In addition, the user can designate an ROI of a predetermined size including the cell periphery in a free shape such as a rectangle or a circle. The data acquisition unit 21 outputs the acquired pathological images and the ROI to the search unit 22.

The search unit 22 searches for other regions similar to each ROI from the pathological images based on the pathological images and the ROI input from the data acquisition unit 21. Then, the search unit 22 outputs the search result to the output unit 23.

For instance, the search unit 22 divides the pathological image into partial images of a plurality of ROI sizes. Then, the search unit 22 extracts features from the ROI and the partial images, respectively, and maps the extracted features onto a feature space. The search unit 22 calculates a degree of similarity based on the distance between the ROI and the partial images in the feature space. The search unit 22 determines whether the ROI and the partial images are similar based on the degree of similarity. For instance, the search unit 22 expresses the degree of similarity as a numerical value from 0 to 100, and if the degree of similarity is equal to or greater than a predetermined threshold, it determines that the ROI and the partial images are similar. On the other hand, if the degree of similarity is less than a predetermined threshold, the search unit 22 determines that the ROI and the partial images are not similar.

Note that the search unit 22 performs the above processing for each type of feature. Types of features include a cell morphology, a cell nucleus size, a cell nucleus shape, and the like. For instance, in a case where the cell morphology is used as the type of feature, the search unit 22 extracts the features of the cell morphology from the ROI and the partial images, respectively, and calculates the degree of similarity. Then, the search unit 22 determines whether the cell morphology included in the ROI and the cell morphology included in the partial images are similar based on the degree of similarity.

In response to detection of one or more other regions within the pathological image that are similar to the ROI, namely, partial images similar to the ROI (hereinafter also referred to as "similar images"), the search unit 22 outputs information regarding the similar images to the output unit 23. Specifically, the search unit 22 outputs the pathological image including the similar image, positional information such as coordinates of the similar image in the pathological image, and the degree of similarity between the ROI and the similar image to the output unit 23.

The output unit 23 generates display data based on the information regarding the similar image input from the search unit 22, and outputs the generated display data to the display unit 16.

In the above configuration, the data acquisition unit 21 corresponds to examples of an image acquisition means and an ROI acquisition means, the search unit 22 corresponds to an example of a search means, and the output unit 23 is an example of an output means.

### [Display Example]

Next, a display example of the search result by the display unit 16 according to the first example embodiment will be described.

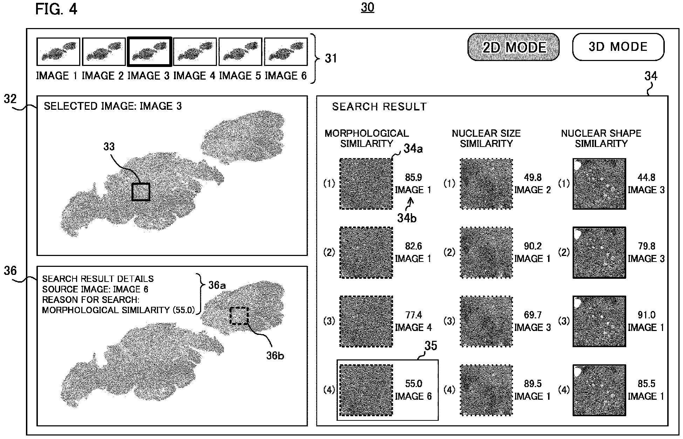

FIG. 4 illustrates an example of a search result display. In the display example illustrated in FIG. 4, a display region 30 includes a pathological image region 31, a selected image 32, an ROI 33, a search result region 34, a cursor 35, and detailed information 36.

The pathological image region 31 is a region for displaying the pathological image input to the pathology diagnosis support device 100. In FIG. 4, six serial section pathological image specimens are displayed in the pathological image region 31. The selected image 32 is a pathological image selected from the pathological image region 31 by an operation of a user. In FIG. 4, "IMAGE 3" is displayed as the selected image 32. The ROI 33 indicates a region to be searched. The ROI 33 is designated from the selected image 32 by the operation of a user.

The search result region 34 is a region for displaying search results. As illustrated in FIG. 4, a plurality of search results are displayed in the search result region 34 for each type of feature. The plurality of search results include a similar image and information regarding that similar image. The information regarding the similar image includes the degree of similarity between the ROI and the similar image, and a file name of the pathological image including the similar image (a pathological image serving as a search source). For instance, a search result (1) for a "morphological similarity" displays a similar image 34a and information 34b regarding the similar image. From the information 34b regarding the similar image, the user can understand that the degree of similarity of the cell morphology included in the ROI and the similar image is "85.9", and the pathological image serving as the search source is "IMAGE 1".

The cursor 35 is for selecting a result of interest among the search results displayed in the search result region 34, and can be selected by an operation of the user. In the example illustrated in FIG. 4, the user has operated the input unit 17 to position the cursor 35 on a search result (4) for the "morphological similarity." The detailed information 36 indicates a region for displaying details of the search result selected by the cursor 35. The detailed information 36 displays the pathological image serving as the search source and the information regarding the similar image. In addition, a region similar to the ROI (hereinafter also referred to as a "similar region") is superimposed on the pathological image serving as the search source. The similar region of the ROI is generated based on the positional information of the similar image in the pathological image. In the example illustrated in FIG. 4, the detailed information 36 displays the pathological image serving as the search source and information 36a regarding the similar image. Also, a similar region 36b of the ROI 33 is superimposed on the pathological image serving as the search source. The detailed information 36 enables the user to identify a position of the similar region of the ROI in the pathological image serving as the search source.

In the search result region 34 illustrated in FIG. 4, four search results are displayed for each type of feature, but if there are four or more search results, the four or more search results may be displayed depending on a space in the search result region 34. In addition, search results having a high degree of similarity may be preferentially displayed.

In the search result region 34 illustrated in FIG. 4, three types of features are displayed, but if there are three or more types of features, three or more types of features may be displayed depending on the space in the search result region 34. In addition, only a specific type of feature may be displayed by an operation of the user.

Furthermore, in FIG. 4, the similar region 36b is indicated by a rectangle, but the size and shape of the similar region 36b may be changed according to the size and shape of the ROI 33.

### (Display Example 2)

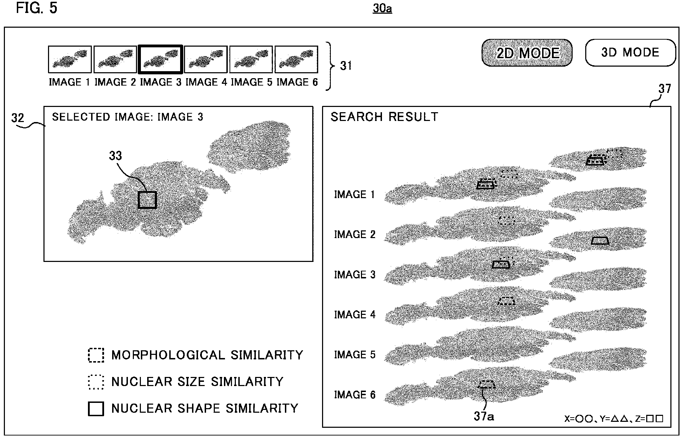

FIG. 5 illustrates another display example of the search result. FIG. 5 illustrates a display mode of the search result that differs from that illustrated in FIG. 4. In FIG. 5, the pathology diagnosis support device 100 performs a three-dimensional image processing by overlapping respective pathological images of sections of the serial section pathological image specimen which has been input as the pathological image at predetermined intervals. Then, the pathology diagnosis support device 100 displays the similar regions of the ROI by superimposing the similar regions on the respective pathological images of the sections.

Specifically, in the example illustrated in FIG. 5, a display region 30a includes the pathological image region 31, the selected image 32, the ROI 33, and a search result region 37. The pathological image region 31, the selected image 32, and the ROI 33 are similar to those as in FIG. 4, and thus, explanations thereof will be omitted.

In the search result region 37, the respective pathological images of the sections are displayed in a three-dimensional manner by being superimposed at predetermined intervals. In addition, each similar region of the ROI 33 is superimposed on each of the respective pathological images of the sections. The display mode of the similar region differs depending on the type of feature. In the example illustrated in FIG. 5, for the morphological similarity, the similar region is displayed as a dashed rectangle; for nuclear size similarity, the similar region is displayed as a dotted rectangle; and for nuclear shape similarity, the similar region is displayed as a solid rectangle. For instance, by this display of a similar region 37a, it is possible for the user to understand that there is a region in "IMAGE 6" that has a morphology similar to the ROI 33. Furthermore, the pathological images displayed in the search result region 37 can be displayed from various angles by operations of the user. By such the display as illustrated in FIG. 5, it is possible for the user to recognize a positional relationship between a similar region included in one pathological image and a similar region included in another pathological image.

Note that in the search result region 37 illustrated in FIG. 5, three types of features are displayed, but in a case where there are three or more types of features, the three or more types of features may be displayed depending on a space in the search result region 37. In addition, only specific type of feature may be displayed by an operation of the user.

Furthermore, in FIG. 5, the similar region is indicated by the rectangle, but the size and shape of the similar region may be changed according to the size and shape of the ROI 33.

### (Display Example 3)

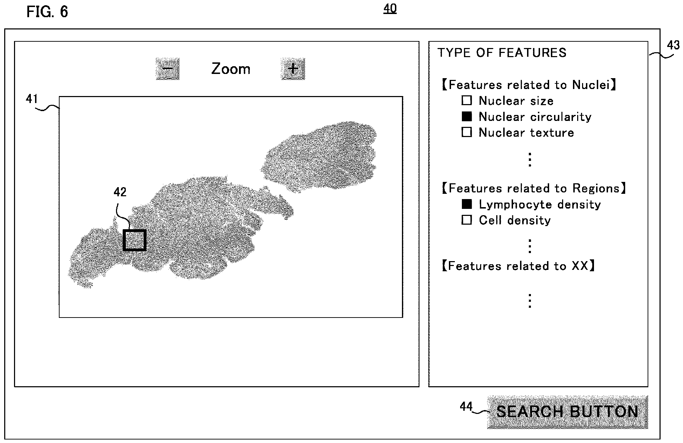

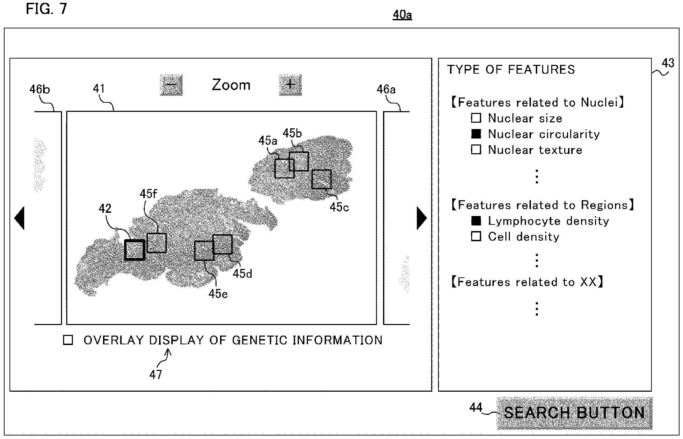

FIG. 6 and FIG. 7 illustrate other display examples of the search screen and search results.

FIG. 6 illustrates an example of the search screen. In the display example illustrated in FIG. 6, a search screen 40 includes a pathological image 41, an ROI 42, a feature type selection region 43, and a search button 44.

The pathological image 41 is the pathological image input to the pathology diagnosis support device 100. The ROI 42 indicates a region to be searched. The ROI 42 is designated in the pathological image 41 by an operation of the user. The feature type selection region 43 is a region for selecting the type of feature. The type of feature is selected by an operation of the user. In the feature type selection region 43 illustrated in FIG. 6, as examples of feature types, a nuclear size, a nuclear circularity, a nuclear texture, a lymphocyte density, and a cell density are displayed. In the example illustrated in FIG. 6, the "nuclear circularity" and the "lymphocyte density" are selected by the user. In a case where the user inputs the ROI 42 and the type of feature, and presses the search button 44, the pathology diagnosis support device 100 searches for the similar image of the ROI 42 in the pathological image based on the type of feature selected by the user.

FIG. 7 illustrates a display example of the search result in FIG. 6. In the display example illustrated in FIG. 7, a search result screen 40a includes the pathological image 41, the ROI 42, the feature type selection region 43, the search button 44, search results 45a to 45f, other pathological images 46a and 46b, and the genetic information overlay 47. Note that the pathological image 41, the ROI 42, the feature type selection region 43, and the search button 44 are similar to those in FIG. 6, and thus, explanations thereof will be omitted.

The search results 45a to 45f correspond to the similar regions of the ROI 42. In FIG. 7, the search results 45a to 45f are superimposed on the pathological image 41, indicating that the search results 45a to 45f correspond to regions similar to the ROI 42 with respect to the "nuclear circularity" and the "lymphocyte density."

The other pathological images 46a and 46b are pathological images different from the pathological image 41. In a case where there are a plurality of pathological images, the pathological images can be switched and displayed by a slide operation of the user or the like. The genetic information overlay 47 is an item for selecting whether to display results of the spatial gene expression analysis. In a case where genetic information overlay 47 is selected, the results of the spatial gene expression analysis are superimposed on the pathological image being displayed.

### [Similar Image Search Process]

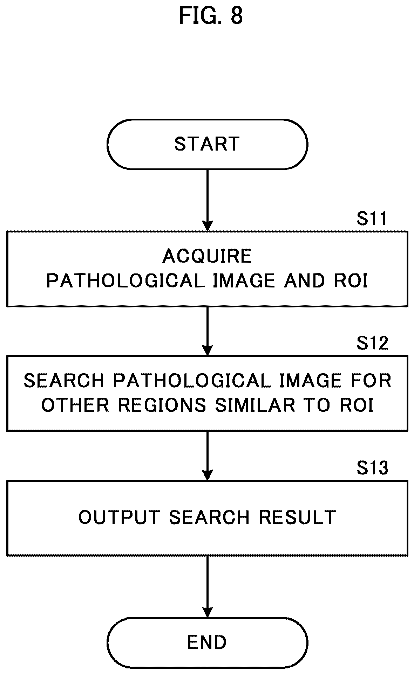

Next, a similar image search process for performing the above similar image search will be described. FIG. 8 is a flowchart of the similar image search process by the pathology diagnosis support device 100. This similar image search process is realized by the processor 12 illustrated in FIG. 2 executing a program prepared in advance and functioning as the respective elements illustrated in FIG. 3.

First, the data acquisition unit 21 acquires the pathological image and the ROI. The data acquisition unit 21 outputs the pathological image and the ROI, which have been acquired, to the search unit 22 (step S11). Next, the search unit 22 searches for other regions similar to the ROI in the pathological image based on the pathological image and the ROI, which have been input from the data acquisition unit 21, and outputs one or more search results to the output unit 23 (step S12). Next, the output unit 23 generates display data based on the one or more search results input from the search unit 22, and outputs the display data to the display unit 16 (step S13). After that, the similar image search process is terminated.

### [Modifications]

Next, modifications of the first example embodiment will be described. The following modifications can be appropriately combined and applied to the first example embodiment.

### (Modification 1)

In the first example embodiment, the pathology diagnosis support device 100 searches for other regions similar to an ROI from a pathological image, but the application of the present disclosure is not limited thereto. For instance, the pathology diagnosis support device 100 may calculate the degree of similarity between ROIs based on two or more ROIs designated by the user.

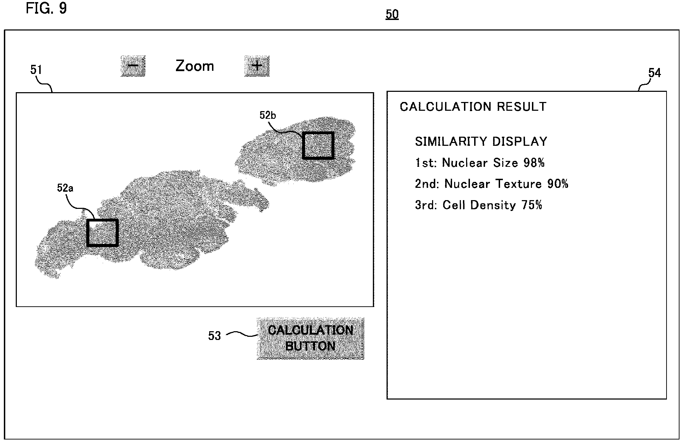

FIG. 9 illustrates a display example of Modification 1. In the display example of Modification 1, a similarity calculation screen 50 includes a pathological image 51, ROIs 52a and 52b, a calculation button 53, and a calculation result region 54.

The pathological image 51 is the pathological image input to the pathology diagnosis support device 100. The ROIs 52a and 52b indicate respective regions for which the degree of similarity is to be calculated. The ROIs 52a and 52b are designated in the pathological image 51 by an operation of the user. In a case where the user designates the ROIs 52a and 52b in the pathological image 51 and presses the calculation button 53, the pathology diagnosis support device 100 calculates the degree of similarity between the ROIs for each type of feature. The calculation result region 54 is a region for displaying calculation results by the pathology diagnosis support device 100. As illustrated in FIG. 9, the calculation result region 54 displays the calculation results of the degree of similarity for each type of feature. Note that in the calculation result region 54 illustrated in FIG. 9, top three calculation results are displayed in descending order of the degree of similarity, but three or more calculation results may be displayed depending a the space in the calculation result region 54. By the display as illustrated in FIG. 9 it is possible for the user to recognize the degree of similarity between the ROIs designated by the user.

Note that in the example illustrated in FIG. 9, the user designates two ROIs, but the user can also designate three or more ROIs. For instance, the pathology diagnosis support device 100 extracts two ROIs from among a plurality of ROIs designated by the user, and calculates the degree of similarity between the ROIs. The pathology diagnosis support device 100 calculates each degree of similarity for all combinations of ROIs, and calculates an average value of degrees of similarity. After that, the pathology diagnosis support device 100 displays the calculated average value as the degree of similarity between the plurality of ROIs designate by the user in the calculation result region 54.

### (Modification 2)

The ROIs and the similar regions of the ROIs can be used as training data for a machine learning model. For instance, the user selects the ROIs and the similar regions of the ROIs that the user desires to use as the training data. Then, the user assigns labels, such as a type of lesion, with respect to the ROIs and the similar regions of the ROIs, and saves the labels in the pathology diagnosis support device 100. At this time, the pathology diagnosis support device 100 may collectively assign the labels to the similar regions retrieved from the same ROI. By saving the similar regions of the ROIs as the training data in this manner, it becomes possible to efficiently collect the training data.

### <Second Example Embodiment>

Next, a second example embodiment of the present invention will be described. A system configuration, a hardware configuration, and a functional configuration of a pathology diagnosis support device according to the second example embodiment are similar to those of the pathology diagnosis support device 100 according to the first example embodiment, but to distinguish the pathology diagnosis support device from the pathology diagnosis support device 100, the pathology diagnosis support device according to the second example embodiment will hereinafter be referred to as a pathology diagnosis support device 100x. The pathology diagnosis support device 100x according to the second example embodiment differs from the pathology diagnosis support device 100 according to the first example embodiment in that it is possible to search for other regions similar to the ROI from the pathological images of patients other than the same patient. The system and hardware configurations of the pathology diagnosis support device 100x according to the second embodiment are similar to those of the device 100 in the first embodiment, and will not be described in detail.

### [Functional Configuration]

The functional configuration of the pathology diagnosis support device 100x according to the second example embodiment is similar to that of the pathology diagnosis support device 100 according to the first example embodiment. In addition, the search unit 22 and the output unit 23 operate in a similar manner as in the pathology diagnosis support device 100 according to the first example embodiment, and thus will not be described in detail.

The pathological images and the ROIs are input to the pathology diagnosis support device 100x via the I/F 11. The pathological images include an observation target pathological image (hereinafter, also referred to as an "observation target image"), and a pathological image of a search target (hereinafter, also referred to as a "search target image"). The observation target image is a pathological image observed by the user, such as the pathological image subject to the pathological diagnosis. The search target image is an image to be searched. The ROI is designated by the user from the observation target image.

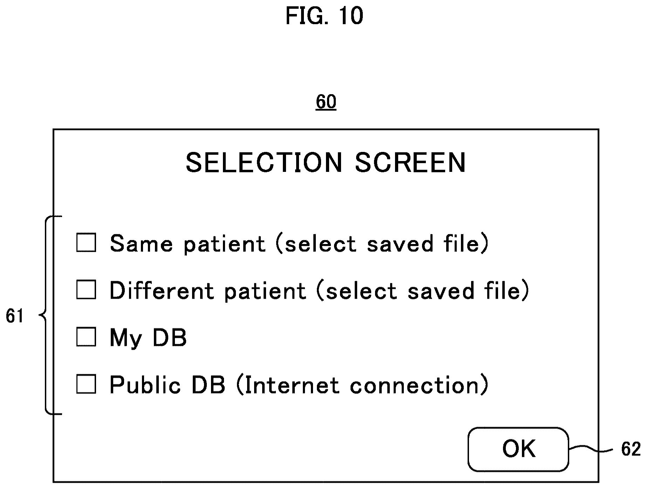

Note that in the pathology diagnosis support device 100x of the present example embodiment, the user can select the pathological image to be used as the search target image. FIG. 10 illustrates an example of a selection screen for the search target image. A selection screen 60 is displayed on the display unit 16 of the pathology diagnosis support device 100x. The selection screen 60 includes a selection region 61 and an OK button 62. The selection region 61 is a region for selecting the search target image. In FIG. 10, examples of search target images such as the pathological images of the same patient, the pathological images of other patients, a MY database, and a public database are displayed. In a case where the user selects the search target image from the selection region 61 and presses the OK button 62, the pathology diagnosis support device 100x acquires the selected search target image from an external device and the like. By this manner, it is possible for the user to use the pathological images of patients other than the same patient as the search target images.

The observation target image, the search target image, and the ROI are input to the data acquisition unit 21. The data acquisition unit 21 outputs the observation target image, the search target image, and the ROI, which have been acquired, to the search unit 22. The search unit 22 searches for other regions similar to the ROI from the search target image based on the ROI and the search target image input from the data acquisition unit 21. Then, the search unit 22 outputs one or more search results to the output unit 23. The output unit 23 generates the display data based on the one or more search results input from the search unit 22 and outputs the display data to the display unit 16.

### [Display Examples]

Next, display examples by the display unit 16 according to the second example embodiment will be described.

### (Display Example 1)

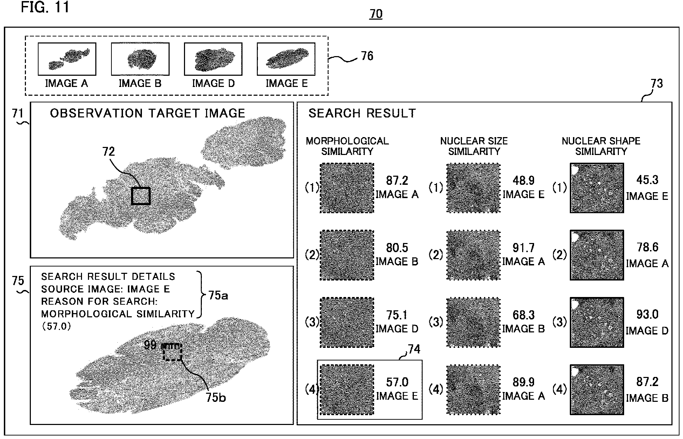

FIG. 11 illustrates a display example for search results in a case where the pathological images other than those of the same patient are used as the search target images. In the display example illustrated in FIG. 11, a display region 70 includes an observation target image 71, an ROI 72, a search result region 73, a cursor 74, detailed information 75, and a similar region display region 76.

The observation target image 71 is the observation target image input to the pathology diagnosis support device 100x. The ROI 72 indicates a region to be searched by the user. The ROI 72 is designated by an operation of the user from the observation target image 71.

The search result region 73 is a region for displaying search results. As illustrated in FIG. 11, in the search result region 73, a plurality of search results are displayed for each type of feature. The search results include a similar region and information concerning that similar region. The information concerning the similar region includes the degree of similarity between the ROI and the similar region, and a file name of the pathological image (the pathological image of the search source) including the similar region. The cursor 74 is used to select a result of interest among the search results displayed in the search result region 73, and the user can select the result of interest by an operation. In the example illustrated in FIG. 11, the user operates the input unit 17 to position the cursor 74 to the search result (4) of the "morphological similarity". The detailed information 75 is a region for displaying details of the search result selected by the cursor 74. The detailed information 75 displays the pathological image of the search source and information concerning the similar region. In addition, the region similar to the ROI is superimposed on the pathological image of the search source. In the example illustrated in FIG. 11, the detailed information 75 displays the pathological image of the search source and information 75a regarding the similar region. In addition, a similar region 75b of the ROI 72 is superimposed on the pathological image of the search source. By the detailed information 75, it is possible for the user to identify a position of the similar region of the ROI in the pathological image of the search source.

In the similar region display region 76, search target images including the similar regions from among a plurality of search target images are displayed. The similar region display region 76 is displayed after a similar region search process by the pathology diagnosis support device 100x.

Note that in the search result region 73 illustrated in FIG. 11, four search results are displayed for each type of feature, but if there are four or more search results, the four or more search results may be displayed depending on a space in the search result region 73. In addition, search results having a high degree of similarity may be preferentially displayed.

Furthermore, in the search result region 73 illustrated in FIG. 11, three types of features are displayed, but if there are three or more types of features, the three or more types of features may be displayed depending on the space in the search result region 73. In addition, only specific type of feature may be displayed by an operation of the user.

Furthermore, in FIG. 11, the similar region 75b is illustrated as a rectangle, but the size and shape of the similar region 75b may be changed according to the size and shape of the ROI 72.

Moreover, in the similar region display region 76 illustrated in FIG. 11, four similar regions are displayed, but if there are four or more similar regions, the four or more similar regions may be displayed depending on a space in the similar region display region 76. In addition, the similar regions with a high degree of similarity may be preferentially displayed.

### (Display Example 2)

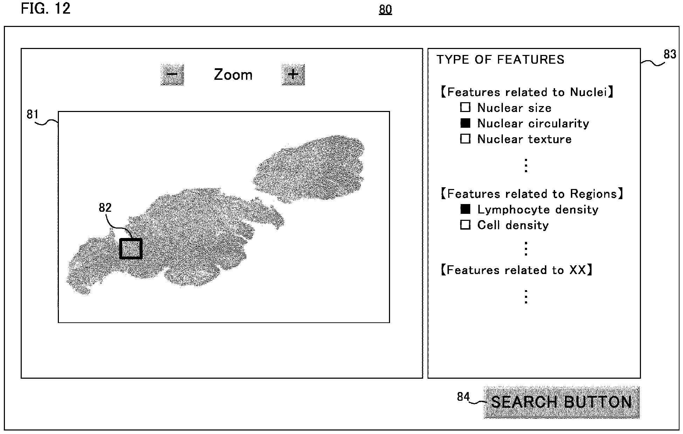

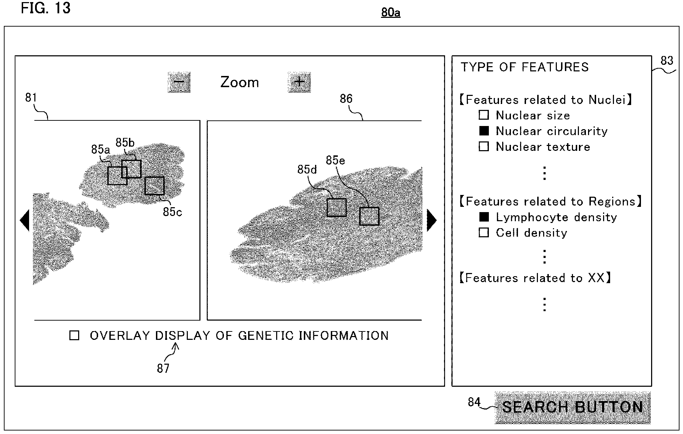

FIG. 12 and FIG. 13 illustrate other display examples of the search screen and search results.

FIG. 12 illustrates an example of the search screen. In the display example illustrated in FIG. 12, a search screen 80 includes a pathological image 81, an ROI 82, a feature type selection region 83, and a search button 84. The search screen 80 in FIG. 12 is similar to the search screen 40 in FIG. 6, and an explanation thereof will be omitted.

Now, in the example illustrated in FIG. 12, the ROI 82 is designated in the pathological image 81. Note that the pathological image 81 is the observation target image. Also, the "nuclear circularity" and the "lymphocyte density" are selected as the types of features. In a case where the user presses the search button 84, the pathology diagnosis support device 100x searches for regions similar to the ROI 82 in the pathological image 81 and the search target image based on the types of features.

FIG. 13 illustrates a display example for the search results in FIG. 12. In the display example illustrated in FIG. 13, a search result screen 80a includes a pathological image 81, a feature type selection region 83, a search button 84, search results 85a to 85e, a search target image 86, and a genetic information overlay 87. Note that the search results 85a to 85e are simply referred to as "search results 85" unless individually distinguished.

The search results 85a to 85c are regions similar to the ROI 82 designated in FIG. 12. In FIG. 13, the search results 85a to 85c are superimposed on the pathological image 81, indicating that the search results 85a to 85c are regions with the "nuclear circularity" and the "lymphocyte density" similar to the ROI 82 designated in FIG. 12.

The search target image 86 is a search target image that includes the search results 85. The user can switch the display from the pathological image 81 to the search target image 86 by a slide operation or the like. Also, in a case where there are a plurality of search target images that include the search results 85, the user can switch and display the search target images by the slide operation or the like. In FIG. 13, the search results 85d and 85e are superimposed on the search target image 86, indicating that the search results 85d and 85e are regions with the "nuclear circularity" and the "lymphocyte density" similar to the ROI 82 designated in FIG. 12. The genetic information overlay 87 is an item for selecting whether or not to display results of the spatial gene expression analysis. In a case where the genetic information overlay 87 is selected, the results of the spatial gene expression analysis can be superimposed on the pathological image 81 and the search target image 86.

Note that in FIG. 12 and FIG. 13, the pathology diagnosis support device 100x searches for regions similar to the ROI 82 in the pathological image 81 and the search target image, and displays the results, but the pathology diagnosis support device 100x may search for regions similar to the ROI 82 alone from the search target image, and display the results.

### [Modifications]

Next, modifications of the second example embodiment will be described. The following modifications can be appropriately combined and applied to the second example embodiment.

### (Modification 1)

The ROI and regions similar to the ROI can be used as the training data for the machine learning model. For instance, the user selects the ROI and the regions similar to ROIs that the user desires to use as the training data. Then, the user assigns labels, such as the type of lesion, to the ROI and the regions similar to the ROI, and saves the labels in the pathology diagnosis support device 100x. At this time, the pathology diagnosis support device 100x may collectively assign the labels to the similar regions retrieved from the same ROI. By saving the similar regions of the ROIs as the training data in this manner, it becomes possible to efficiently collect the training data.

### <Third Example Embodiment>

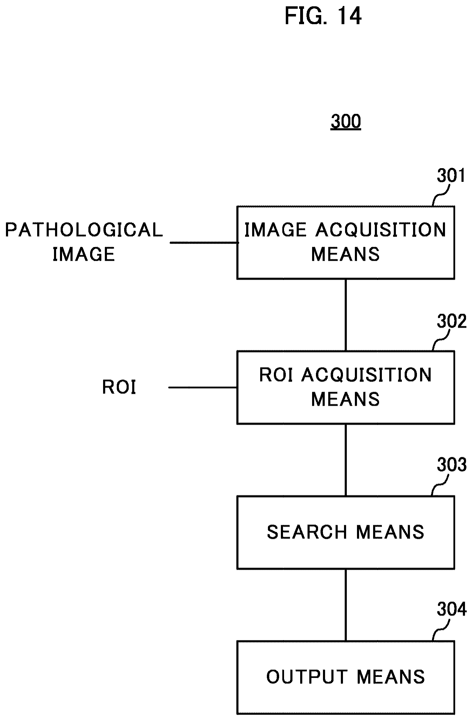

FIG. 14 is a block diagram illustrating a functional configuration of a pathology diagnosis support device according to a third example embodiment. A pathology diagnosis support device 300 includes an image acquisition means 301, an ROI acquisition means 302, a search means 303, and an output means 304.

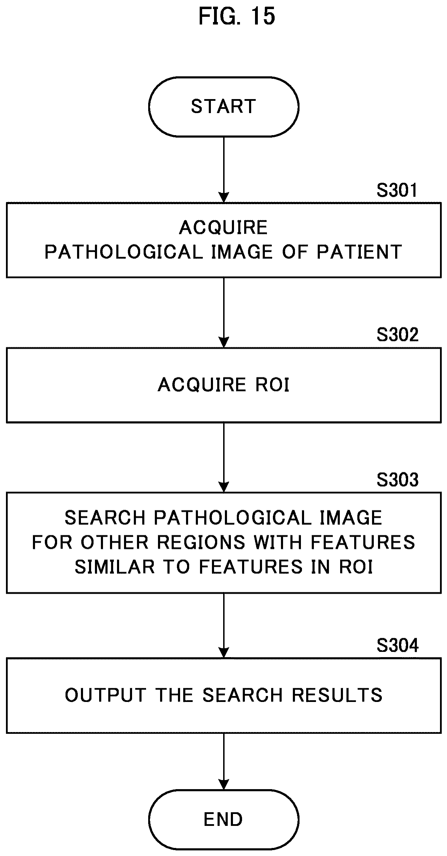

FIG. 15 is a flowchart of processing by the pathology diagnosis support device according to the third example embodiment. The image acquisition means 301 acquires a pathological image of a patient (step S301). The ROI means 302 acquires a region of interest which is selected by the user in the pathological image (step S302). The search means 303 searches for other regions having features similar to the features included in the region of interest in the pathological image (step S303). The output means 304 outputs the search results (step S304).

According to the pathology diagnosis support device 300 of the third example embodiment, it is possible to search for regions in a pathological image that have similar features to a predetermined region.

A part or all of the example embodiments described above may also be described as the following supplementary notes, but not limited thereto.

### (Supplementary note 1)

A pathology diagnosis support device comprising:
an image acquisition means configured to acquire a pathological image of a patient;
an ROI acquisition means configured to acquire a region of interest which is selected by a user from the pathological image;
a search means configured to search, from the pathological image, for other regions having features similar to features included in the region of interest; and
an output means configured to output results of the search,
wherein the search means performs a search for one or more regions of a similar size to the region of interest as search targets, for each type of feature included in the pathological image,
the output means outputs one or more results of the search for each type of feature, and
the results of the search include a reason for the search and information of a search source.

### (Supplementary note 2)

The pathology diagnosis support device according to supplementary note 1, wherein
the pathological image includes a plurality of images related to serial sections of the same cell, and
the output means performs a three-dimensional display process by superimposing the pathological images, and superimposes the results of the search on the pathological images.

### (Supplementary note 3)

The pathology diagnosis support device according to supplementary note 1, wherein
the image acquisition means acquires a pathological image of the patient and pathological images of other patients,
the ROI acquisition means acquires a region of interest which is selected by the user from the pathological image of the patient, and
the search means searches for the other regions having features similar to the features included in the region of interest, from the pathological images of the other patients.

### (Supplementary note 4)

The pathology diagnosis support device according to supplementary note 1, further comprising a feature type acquisition means configured to acquire a type of feature designated by the user,
wherein the search means calculates features depending on the type of feature included in the region of interest, and searches for the other regions having features similar to the calculated features from the pathological image.

### (Supplementary note 5)

The pathology diagnosis support device according to supplementary note 1, further comprising a calculation means, wherein
the ROI acquisition means acquires a plurality of regions of interest which are selected by the user from the pathological image,
the calculation means calculates each degree of similarity between the plurality of regions of interest for each type of feature, and
the output means outputs a calculation result.

### (Supplementary note 6)

The pathology diagnosis support device according to supplementary note 1, further comprising a collection means configured to collect the one or more results of the search,
wherein the collection means collects images of the other regions having features similar to the features included in the pathological image, and uses the images as training data.

### (Supplementary note 7)

An information processing method comprising:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.

### (Supplementary note 8)

A recording medium storing a program, the program causing a computer to perform a process comprising:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.

While the disclosure has been described with reference to the example embodiments and examples, the disclosure is not limited to the above example embodiments and examples. It will be understood by those of ordinary skill in the art that various changes in form and details may be made therein without departing from the spirit and scope of the present disclosure as defined by the claims.

### DESCRIPTION OF SYMBOLS

- 21: Data acquisition unit
- 22: Search unit
- 23: Output unit
- 100: Pathology diagnosis support device

## Claims

1. A pathology diagnosis support device comprising:
an image acquisition means configured to acquire a pathological image of a patient;
an ROI acquisition means configured to acquire a region of interest which is selected by a user from the pathological image;
a search means configured to search, from the pathological image, for other regions having features similar to features included in the region of interest; and
an output means configured to output results of the search,
wherein the search means performs a search for one or more regions of a similar size to the region of interest as search targets, for each type of feature included in the pathological image,
the output means outputs one or more results of the search for each type of feature, and
the results of the search include a reason for the search and information of a search source.

2. The pathology diagnosis support device according to claim 1, wherein
the pathological image includes a plurality of images related to serial sections of the same cell, and
the output means performs a three-dimensional display process by superimposing the pathological images, and superimposes the results of the search on the pathological images.

3. The pathology diagnosis support device according to claim 1, wherein
the image acquisition means acquires a pathological image of the patient and pathological images of other patients,
the ROI acquisition means acquires a region of interest which is selected by the user from the pathological image of the patient, and
the search means searches for the other regions having features similar to the features included in the region of interest, from the pathological images of the other patients.

4. The pathology diagnosis support device according to claim 1, further comprising a feature type acquisition means configured to acquire a type of feature designated by the user,
wherein the search means calculates features depending on the type of feature included in the region of interest, and searches for the other regions having features similar to the calculated features from the pathological image.

5. The pathology diagnosis support device according to claim 1, further comprising a calculation means, wherein
the ROI acquisition means acquires a plurality of regions of interest which are selected by the user from the pathological image,
the calculation means calculates each degree of similarity between the plurality of regions of interest for each type of feature, and
the output means outputs a calculation result.

6. The pathology diagnosis support device according to claim 1, further comprising a collection means configured to collect the one or more results of the search,
wherein the collection means collects images of the other regions having features similar to the features included in the pathological image, and uses the images as training data.

7. An information processing method comprising:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.

8. A recording medium storing a program, the program causing a computer to perform a process comprising:
acquiring a pathological image of a patient;
acquiring a region of interest which is selected by a user from the pathological image;
performing a search, for each type of feature, for other regions in the pathological image that have features similar to those included in the region of interest, using, as search targets, regions having the same size as the region of interest; and
outputting one or more results of the search for each type of feature, and
the results of the search include a reason for the search and information of a search source.
